# EUROPEAN PATENT APPLICATION

(11) **EP 2 868 268 A1**
(43) Date of publication of application: **06.05.2015**
(21) Application number: 13190867.5
(22) Date of filing: 30.10.2013
(51) Int. Cl.: A61B 5/021, A61B 5/022, A61B 5/00

(54) **System for evaluating pain modulation, spatial and/or temporal summation of pain or descending inhibition of pain in a human**

(71) Applicant: NociTech ApS, 3400 Tulstrup (DK)
(72) Inventor: Arendt-Nielsen, Lars, 9000 Aalborg (DK); Graven-Nielsen, Thomas, 9000 Aalborg (DK)
(74) Representative: Jakobsson, Jeanette Helene

(57) **Abstract**

The present invention describes a system for inducing at least compressional or shear force on a subject and evaluating pain modulation, spatial and/or temporal summation of pain, or descending inhibition of pain in the subject, said system comprising a cuff unit comprising an inflatable cuff having at least two chambers or comprising at least two inflatable cuffs having one or more chambers each, each chamber having a coupling for connection to a compressor or other activator device; a compressor or other activator device arranged to provide inflation; and a computer unit with a control function, said control function directed to control of inflation rates and independent control of each chamber. The present invention is also directed to an inflatable cuff for a system according to above and also to the use and methods involving the use of such a system.

## Description

### Field of the invention

The present invention relates to a system for inducing compressional or shear force on a subject and evaluating spatial and/or temporal summation of pain, or descending inhibition of pain in the subject, to a cuff intended for such a system, use of such a system and to methods involving the use of such a system. The present invention is e.g. directed to areas involving pain research, pain management, diagnosing, stratification of patients, and/or development of pain treatments, such as drugs and devices.

### Technical Background

In WO2011/103880 there is disclosed a device for evoking a temporal summation of pain in a subject. The device comprises a stimulation element (SE) arranged generating a physical stimulation on a skin surface area of at least 20 cm² according to a control signal (CS), where the stimulation (PS) evokes pain in the subject. A processor (PR) generates the control signal (CS) to the stimulation element (SE) so as to provide a repetitive stimulation pattern comprising at least repetitions of: a) applying stimulation for a period (SP) of 0.5-120 seconds, b) stopping stimulation for a period (TP) of 0.5-20 seconds. The stimulation element may include an inflatable tourniquet arranged for providing a compressional stimulation of an arm or a leg. The device is suited for providing a measure of temporal summation in a subject with different intensities, and this measure can be used for determining if the subject suffers from central nervous sensitization which is an important diagnosis with respect to effective analgesic treatment. In some embodiments, the device is arranged to collect pain responses during the stimulation and to calculate a measure of temporal summation of the subject accordingly, e.g. in the form of a rate of pain change (VAS) versus time (T).

One aim of the present invention is to provide an improved system for measuring and evaluating spatial and/or temporal summation of pain or descending inhibition of pain in a subject, which system provides increased adjustability of the system with reference to what to assess and how to assess.

### Summary of the invention

The stated purpose above is achieved by a system for inducing at least compressional or shear force on a subject and evaluating pain modulation, spatial and/or temporal summation of pain or descending inhibition of pain in the subject, said system comprising:
- a cuff unit comprising an inflatable cuff having at least two chambers or comprising at least two inflatable cuffs having one or more chambers each, each chamber having a coupling for connection to a compressor or other activator device;
- a compressor or other activator device arranged to provide inflation; and
- a computer unit with a control function, said control function directed to control of inflation rates and independent control of each chamber.

As disclosed above, the cuff unit in the system according to the present invention may either comprise one inflatable cuff having at least two separate chambers or several inflatable cuffs having one or more separate chambers. This feature of the system according to the present invention is a requirement to provide independent control of each chamber. The independent control obtained by a system according to the present invention is not provided by a system disclosed in WO2011/103880. Although WO2011/103880 discloses a tourniquet which may comprise multiple inflatable chambers, there is no hint in WO2011/103880 with respect to the control of these chambers. As further discussed below, this independent control may imply different types of control and may also focus on different parameters. Moreover, the independent control according to the present invention may be directed to controlling individual chambers in one and the same cuff or chambers arranged in different cuffs, such as e.g. one cuff used on an arm and e.g. one cuff used on a leg of a patient. In relation to this it is important to realize that the system and cuff unit(s) according to the present invention may be used at any place on the body where the device can be placed and mediate stimuli used for the measurement, such as e.g. fingers, hands, toes, feet, hip, trunk, head and back. This is further discussed below.

It may further be said that the inflation rates used according to the present invention normally are inflation rates of up to 100 kPa/seconds which implies inflation times in the range of from less than a second to minutes.

With respect to the expression "inflation" and with reference to the present invention this implies volume expansion caused by air, gas, water, oil, mechanical expansion, or a combination thereof. Furthermore, the inflatable cuff according to the present invention is arranged to provide pressure to a part of the body target by any kind of inflation. The chamber(s) of the inflatable cuff according to the present invention are connected to a compressor or other activator means (device) to provide for such inflation.

Moreover, besides the control function, the computer unit also has several other functions and/or units normally existing in a computer. Possible examples thereof are measurement and/or analysis and/or display/communication function(s).

### Specific embodiments of the invention

Below specific embodiment of the present invention are provided.

According to one specific embodiment, at least one inflatable cuff of the system has more than two individual chambers. A cuff according to the present invention may in fact have several chambers, also chambers which individually have different size, shape or comprise different material and/or stimulation means when compared to one another. This is also further discussed below.

According to one embodiment of the present invention, independent control of each chamber is independent inflation control and control of each chamber based on pressure, volume, tissue stress and/or tissue strain in a single inflation manner, in a repeated inflation manner and/or in an individual cuff manner (each cuff as a whole or the individual chambers one by one in one given cuff).

According to yet another embodiment, the inflatable cuff has several chambers of which one most distal chamber and one most proximal chamber are mounting chambers and one or more inner chamber(s) are stimulation chamber(s) or other activators integrated in the cuff, such as e.g. activator units directed to electrical, temperature, vibration, different shapes of mechanical probes attached to the cuff(s) or other sensory stimulations, applied individually or in combination. This is one example where the chambers are intended for different purposes. The two outermost chambers are intended to secure correct mounting. It should also be said that the extension of the chambers as such may vary according to the present invention. The chambers may be provided one by one from a long side to the other long side of the cuff, i.e. when being used along an extremity of a patient, or from a short side to the other short side of the cuff, i.e. around the extremity of a patient. Both alternatives and also other alternatives are possible according to the present invention.

Furthermore, as understood from above, the inflatable medium used is e.g. air, however also other types are possible, such as fluids, e.g. water and oil. Moreover, mechanical inflation may be interpreted as any kind of tissue pressure medicated mechanically, directly or indirectly, such as e.g. cylinders placed side by side that can be controlled individually, or mechanical bend that mediate pressure by change length, or a hard shell that are being pressed together and in that way increase the pressure against the tissue it circumvent. Both static and vibrating variants are possible.

The present invention is also directed to an inflatable cuff intended for a system disclosed above. According to one embodiment, there is provided an inflatable cuff for inducing at least compressional or shear force on a subject and evaluating pain modulation, spatial and/or temporal summation of pain or descending inhibition of pain in the subject, said cuff comprising at least two separate chambers, wherein each chamber has a coupling for connection to a compressor or other activator device, and wherein each chamber is arranged for individual inflation and control thereof.

As hinted above, the shape, size, material thereof etc. of the cuff according to the present invention may have many different variations. According to one embodiment, the cuff has at least a portion being made of a material which ensures compliance to adapt to anatomical structures. This may be of interest to ensure a flexible cuff which ensures an evenly tight compression around for instance an arm or a leg, also in the directions where e.g. the endoskeleton is provided on the inside. Furthermore, it may also be of interest to adapt the actual chamber and cuff material for methods where such hard parts inside of an extremity of a patient is affecting the results. The cuff or chamber material may as such be adapted to either measure the effect of such portions or in fact be able to eliminate the contribution of such portions.

The inflatable cuff or portions thereof, such as one chamber or more, according to the present invention may as such be made of a custom-made material which is adapted to anatomical structures and which ensures compliance of the material so that it is matched to the intended stimulus region and stimulus purpose. The material may e.g. be elastic, non-elastic and have different degrees of compliance. According to one embodiment, the material has thixotropic properties.

As mentioned above, the chamber stimulation is directed to at least compressional or shear force, but also other alternatives are possible as added features to one and the same chamber or in fact as something added as a means to an entire cuff or a single chamber thereof. The added stimulation modalities may either be properties incorporated directly into the cuff or may be advices being part of the cuff or outside the cuff but part of the system as stand-alone units. Other stimulus modalities possible according to the present invention are electrical, mechanical, vibration, thermal and chemical stimulation modalities. It should also be mentioned that the actual cuff material may be electrically or thermally conducting according to one embodiment of the present invention.

In relation to the cuff stimulation is may further be said that the actual stimulation may be performed simultaneously, semi-synchronised or in sequence for different alternatives used in a system according to the present invention.

In relation to the shape of the cuff it should be said that the cuff may be adapted for any part of a human body, such as an arm, a leg, feet, toes, one or several fingers, the head or the back, hip etc. Moreover and as said, the extension of the chambers of the cuff may have the direction of both around the extremity or along the extremity, or any variation in-between or combination hereof. All versions are possible. Fact is that a system according to the present invention may comprise several alternatives, both in terms of body structure to evaluate, such as both leg and arm, and also in terms of extension of the actual chambers. This may be of interest to be able to evaluate in many different ways on one and the same patient in the same system.

Moreover, the present invention is also directed to methods for pain assessment. According to one specific embodiment the present invention provides a method for inducing at least compressional or shear force on a subject and evaluating pain modulation, such as spatial and/or temporal summation of pain, or descending inhibition of the pain in the subject, said method comprising applying compressional or shear force pulses to the subject with a force pattern being uniform for pulses in the system according to the present invention. In relation to the method disclosed above and other methods below it is important to understand that the cuff unit may comprise one cuff having several chambers or several cuffs having only one or several chambers. The common feature is, however, the independent control of the at least two chambers.

As hinted above, the cuffs or chambers of the cuffs according to the present invention may be inflated simultaneous or in predefined sequences according to the present invention.

According to yet another embodiment there is provided a method for inducing at least compressional or shear force on a subject and evaluating pain modulation, spatial and/or temporal summation of pain, or descending inhibition of the pain in the subject, said method comprising applying compressional or shear force pulses to the subject with an increasing force pattern over time via the inflatable cuff(s) in the system according to the present invention. In relation to the pulse definitions above it may further be said to both increasing and decreasing force paradigms are possible according to the present invention. Moreover, the actual expression "pulse" should only be seen as an indication that there is not a constant force or constant increase or decrease of force during the entire method and evaluation. A pulse indicates an increase of force which after its force peak is reached transforms into a decrease of force after a certain time. As hinted below, it is important to realize that the system according to the present invention may also be used in methods where a continuous stimulation is applied, implying a fixed stimulus.

Furthermore, provided is also a method for inducing at least compressional or shear force on a subject and evaluating pain modulation, spatial and/or temporal summation of pain, or descending inhibition of the pain in the subject, said method directed to assessing pain control in the subject, and wherein two separate cuffs in a system according to the present invention are used, one or more cuff(s) on one site (e.g. arm or leg) of a patient and the other one or more cuff(s) on another site (e.g. the other arm or leg) of the patient, and wherein one or more of the cuffs are used to induce at least compressional or shear force on the subject and wherein the other one or more cuffs is used to measure the pain sensitivity and/or spatial/temporal summation of pain or descending inhibition of pain in the subject. As understood from above, this method may be used to evaluate down-regulation of pain in a subject (descending inhibition), but also to evaluate e.g. the temporal summation. The cuffs in this case are controlled individually and in defined sequences.

As said before, the cuff system according to the present invention may be used to evaluate descending inhibition, and spatial and temporal summation, all being directed to pain control, e.g. pain management, characterisation, diagnosing, stratification, and/or treatment development. Assessing descending pain control is a paradigm where e.g. the arm and leg is stimulated simultaneously and temporal and spatial assessment is the mode where only one extremity of a patient is stimulated. Moreover, when assessing the descending pain modulation it is likely that the cuff to induce pain (e.g. in the arm) is based on alternating stimulations from different chambers (or modalities) and likewise the assessment on the leg should be able to assess spatial and temporal summation of pain from different chambers and also non-pressure modalities. Thus independent control of one or more cuffs each including one or more chambers and/or modalities.

As mentioned above, according to one specific embodiment two or more separate cuffs are used and are inflated simultaneous or in predefined sequences. Moreover, according to yet another embodiment, two or more separate cuffs are used on different regions of the patient and wherein the two or more separate cuffs are controlled individually and in defined inflating sequences. In relation to this it should be said that all possible different body regions, such as arms, legs, feet, toes, head and arm or leg, back and arm, fingers, and all combinations, are possible according to the present invention.

The methods and system according to the present invention provides the possibility of assessing both descending inhibition and temporal summation in the same session, which may be beneficial.

It may further be said that the methods according to the present invention are directed to stimulation which may involve loops comprising first applying stimulation during a certain time and then stopping stimulation during a certain time, but may also involve continuous stimulation.

Furthermore, the methods according to the present invention may involve calculating a measure such as described according to WO2011/103880, i.e. in the form of a rate of pain change versus time, but may also be directed to measuring pressure/force/volume versus time.

Moreover, and also when comparing to WO2011/103880, the skin surface area used according to the present invention may be at least 20 cm², i.e. just as described in WO2011/103880, but the system according to the present invention may comprise much smaller cuffs and in such cases the skin surface area used may be as small as down to e.g. 1 cm².

Moreover, the present invention is also directed to the use of a system according to above for pain assessment. According to one embodiment there is provided use of a system according to above as a tool for diagnosis, for patient categorizing, for patient selecting, for pain treatment planning and/or for drug screening profiling. Depending on the methods and system used, the diagnosis approach may vary. Treatment planning of a patient may e.g. be directed to evaluating what kind of drug treatment(s) is/are needed, if an operation is to be performed or performed in a specific way or prepared by different way. Moreover, drug screening may be performed by use of the system together with the evaluation of different drugs or combination hereof, and the effect thereof. Or relates to optimize a device treatment, such as its stimulation frequency, volume or anatomically directed.

Furthermore, the system according to the present invention may also used to evaluate the effect of different drugs. According to one embodiment, there is provided the use of a system according to the present invention to evaluate the pharmacological pain treatment of a patient.

Despite intensive research activity, chronic pain remains a significant problem in many patients. For several decades, the mainstay of treatment of chronic pain has been provided by opioids, non-steroidal anti-inflammatory drugs (NSAIDs), anticonvulsants, anti-depressants, anaesthetics and combinations thereof. Unfortunately, whilst these drugs do provide meaningful pain relief, they are insufficiently effective in a significant proportion of patients due to sensitisation and often elicit unwanted and sometimes serious side effects. This has prompted pharmaceutical companies to invest substantially in the search for new medicines to treat chronic pain and many new molecules and molecular mechanisms have been tested in the clinic in the last 10-15 years within indications such as neuropathic pain, osteoarthritis (OA) pain, complex regional pain syndrome, trigeminal neuralgia and chronic low back pain. Use of a system according to above can characterise the patient's pain and pain sensitisation and predict which kind of treatment (such as drug and device) or combination hereof that will work best for the patient. This can be used in the development of such treatment to speed it up and/or decrease the number of patients needed in the clinical trials to demonstrate statistical effect, and when the treatment is approved as a formally companying diagnostics or just used to individualise the treatment of the patients.

Pain and sensitization are major issues in patients with osteoarthritis both before and after total knee arthroplasty (TKA) and revision TKA (re-TKA). Widespread sensitization has been demonstrated in patients with pain after re-TKA and highlight the importance of ongoing nociceptive input for the chronification process. This has important implications for planning and predicting the success of future revisions, and precautions should be taken if patients have widespread sensitization.

Moreover, there is also provided the use of a system according to above, for assessing tissue compliance. As such the system or cuff(s) of the system may be used for assessing tissue compliance, e.g. via the fact that vibrating the muscles with the cuff will provide information about the muscles thixotropic features. Based on different tissue models the cuff pressure can be adapted to local/regional differences in tissue compliance and internal structures (bone, etc.). According to the present invention it is possible to use models of the tissue and calculate the stress/force/share forces in the tissue, and then by having several small cuffs which can be inflated individually it is possible to adapt the force production to areas where it is desired to deliver more force to activate specific areas in the tissue (e.g. bone, joint, ligaments, muscles, fascia, skin and/or diseased area).

According to one embodiment of the present invention there is provided the use of a system according to above for assessing descending pain control and assessing temporal/spatial summation in the same system. The possibility of assessing both descending pain control and assessing temporal/spatial summation in the same system is a clear advantage of the present invention when being compared to other systems.

### Examples and description of the drawings

Below there is provided a proof of concept study is integrating a cuff algometry system according to the present invention in the evaluation of severe knee osteoarthritis.

The figures in the drawings, which are further discussed below, exhibit the following:
*Figure 1A**-D:* Mean (+/- SEM) PDTs, PTTs, PTLs and PDTs ratio for double or single cuff increasing stimulation at constant gradient (1 kPa/s). Significant difference is illustrated (*, P<0.05).
*Figure 2A**-B:* Mean (+/- SEM) normalized VAS scores for ten stimuli of sequential stimulation for knee OA patients and age matching healthy controls. Significant differences compared with first stimulus, and between groups are illustrated (*: P < 0.05, #: P<0.05).
*Figure* 3: Mean (+/- SEM) Ratio between PDTs at baseline and during conditioning pain stimulation (painful constant pressure stimulation in the arm).

### INTRODUCTION

Osteoarthritis (OA) is a major cause of disability in the elderly population and has high prevalence in the society as 40% of women and 25% of men aged 60-70 years are diagnosed with OA (1). Central sensitization has been suggested as one of the underlying mechanisms of pain in OA (2). Continuous and intense nociceptive input from the OA affected knee joint may drive central sensitization (3,4). Knee OA patients have lower pain threshold and enhanced central summation that facilitate temporal summation (increased pain response to repeated stimulation) (5).

This proof of concept study is integrating a cuff algometry system, developed at the Center for Sensory-Motor Interaction (Aalborg University), in the evaluation of severe knee osteoarthritis.

### METHODS

### Subjects

The present report include preliminary assessment of data from the patients including so far which is approximately 75% of the number of patients intended: seventy six knee osteoarthritis (OA) patients (forty four females, age range 48-86 yrs old) and forty two age matched controls (twenty two females, age range 53-79 yrs old). The mean body max index (BMI) for the OA patients s 29.2 ± 5.2 kg/cm², while for controls is 26.1 ± 5.2 kg/cm². Patients will undergo to total knee arthroplasty surgery and consequently are considered as severe knee OA patients. Controls had knee pain corresponding to VAS (0-10) at 3 or less and without other long lasting pain problems in the previous year. All participants have been recruited from the North Denmark Region. Written informed consent is obtained from each subject prior to inclusion in the study. The study has been conducted in accordance with the Declaration of Helsinki and approved by the local ethics committee (N-20120015).

### Cuff Algometry for Assessment of the Pain Sensitivity

Pressure detection pain thresholds (PDT) and pain tolerance thresholds (PTT) are recorded by a computer-controlled cuff-algometer (Aalborg University, Denmark) according to the present invention. A digital visual analogue scale (VAS) is also connected to the system. The VAS is sampled at 10 Hz. Zero and ten cm extremes on the VAS are defined as "no pain" and as "maximal pain", respectively.

A system comprising inter alia a 13-cm wide tourniquet cuff with an equal-sized proximal and distal chamber is used in the method. The cuff is wrapped around the lower leg at the level of the heads of the gastrocnemius muscle. Different assessments are performed in order to evaluate the pain sensitivity, spatial and temporal effects due to pain and conditioning pain stimulation. Each condition is repeated three times and a mean of the different parameters is applied in the statistical analysis.

### Protocols

Three different assessments have been performed during this proof of concept study:
- Increasing pressure stimulation at constant gradient: the pressure is increased with a rate of 1 kPa/s and the maximal pressure limit is 100 kPa. The participants use an electronic VAS to rate their pressure-induced pain intensity and a button to release the pressure. The participants are instructed to rate the pain intensity continuously on the electronic VAS from when the pressure is defined as pain (PDT) and to press the pressure release button when the pain is intolerable (PTT). In addition, it is also evaluated the pain intensity scores at pressure tolerance (PTL).
- Repetitive constant stimulation: sequential stimulation consists of 10 pressure stimuli (1-s duration and 1-s rest) at the average between pressure pain threshold and pressure pain tolerance determined in the previous procedure with a double cuff. The participant assesses the pain intensity continuously throughout the test the VAS scale.
- Evaluation of conditioning pain stimulation: two cuffs are simultaneously used. A double cuff on the leg and a single cuff on the arm of the participant or vice versa. The single cuff is inflated at a constant painful stimulation, while the double cuff is slowly inflated in order to obtain pain threshold and tolerance.

### Statistics

The data are presented as means and standard error of the mean (SEM). Pressure detection thresholds (PDTs), pressure tolerances (PTTs) and VAS value at pressure tolerance (PTLs) between patients and controls are analysed with one-way ANOVAs (1ANOVA). Three different 1ANOVA are performed, evaluating single, double cuff and the ratio between single and double cuff pressure threshold. The normalized VAS after each repetitive stimulation is analysed using a repetitive measure ANOVA with within stimulus number (1-10), and between factor experimental groups (patients, control). VAS sum, indicating the sum of VAS at each stimulus between patients and controls is analysed with one-way ANOVAs. The difference between pressure detection threshold with or without painful stimulation in the arm are also analysed with one-way ANOVAs (1ANOVA). P < 0.05 is considered significant.

### RESULTS (a preliminary subset of patients)

### Pain sensitivity

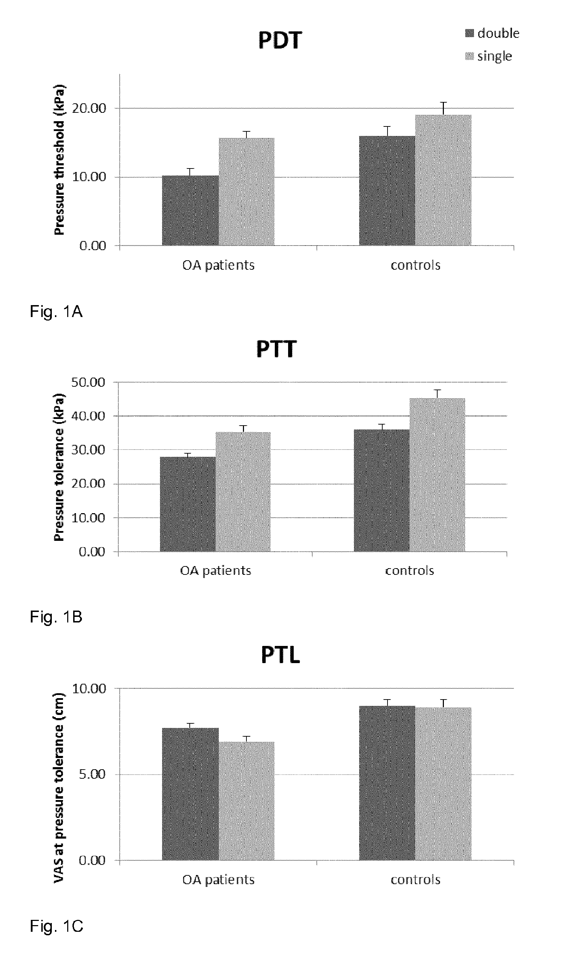

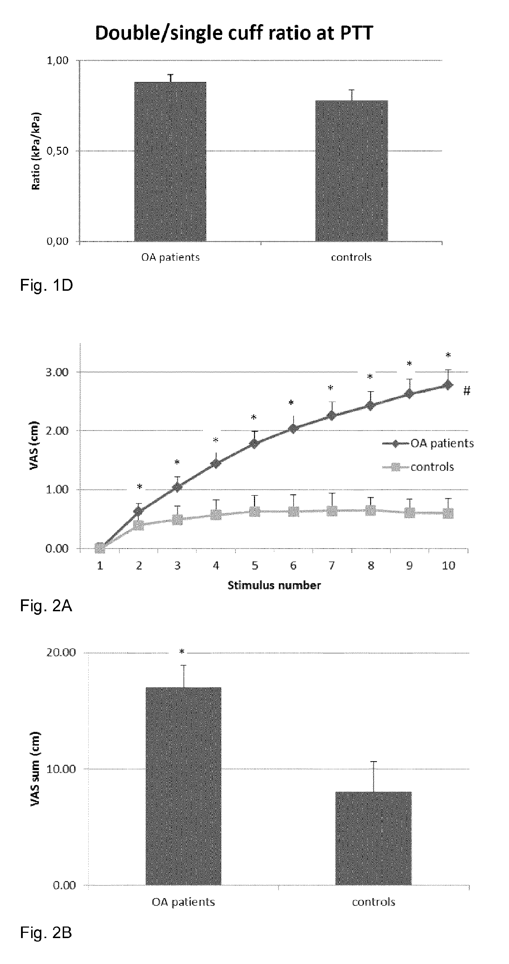

PDTs, PTTs, and PTLs result significantly different between knee OA patients and controls (F_{1,118}<14.8; P<0.03) for both single and double cuff increasing stimulation at constant gradient. Knee OA patients show lower pressure threshold, tolerance and lower VAS value at pressure tolerance compared to age-matched healthy controls. Double cuff assessment resulted in lower threshold and tolerance for both knee OA patients and age-matching healthy controls. The ratio between double and single cuff tolerance thresholds resulted not statistically different between knee OA patients and age-matched healthy controls (F_{1,118}<2.0; P<0.1) (Figure 1). Further analysis may be needed in order to divide all the patients in relation to the gender.

### Temporal summation of pain

A significant increase of normalised VAS scores begins from the 2nd stimulus and continues to 10th stimulus compared with the first stimulus (F_{1,118}=14.1 P < 0.01). This increase is significant different between knee OA patients and healthy controls, as the peak for the first group is 2.77 ± 0.60 cm, while for the second is 0.27 ± 0.25 cm. Similar result regarding the sum of VAS, knee OA patients shows higher sum than control (Figure 2).

### Conditioning Pain Modulation

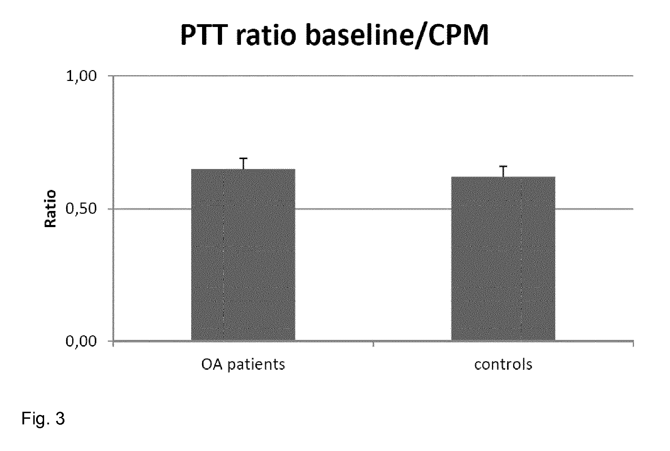

PDTs, PTTs, and PTLs result significantly different between knee OA patients and controls (F_{1,118}<30.8; P<0.01) regarding the conditioning pain stimulation. The ratio between PDTs at baseline and conditioning pain stimulation is not statistically significant, but with a trend showing higher ratio for OA patients in comparison to age-matching healthy controls (Figure 3). This is accordance with previous literature showing reduced conditioning pain stimulation in OA patients.

### Discussion

The cuff algometer results to be a promising tool for evaluating central mechanisms in knee OA patients. Both spatial and temporal components of pain have been evaluated. These preliminary results provide support material for the integration of the cuff algometry technique with commonly used knee OA evaluation techniques.

### References from above

1. Peat G, McCarney R, Croft P. Knee pain and osteoarthritis in older adults: a review of community burden and current use of primary health care. Ann Rheum Dis 2001 ;60(2):91
2. Imamura M, Imamura ST, Kaziyama HH, Targino RA, Hsing WT, de Souza LP, Cutait MM, Fregni F, Camanho GL. Impact of nervous system hyperalgesia on pain, disability, and quality of life in patients with knee osteoarthritis: a controlled analysis. Arthritis Rheum 2008;59:1424-31.
3. Martindale JC, Wilson AW, Reeve AJ, Chessell IP, Headley PM. Chronicsecondary hypersensitivity of dorsal horn neurones following inflammation of the knee joint. Pain 2007;133:79-86.
4. Neugebauer V, Lucke T, Schaible HG. N-Methyl-D-aspartate (NMDA) and non-NMDA receptor antagonists block the hyperexcitability of dorsal horn neurons during development of acute arthritis in rat's knee joint. J Neurophysiol 1993;70:1365-77.
5. Arendt-Nielsen L, Nie H, Laursen MB, Laursen BM, Madeleine P, Simonsen O, Graven-Nielsen T. Sensitization in patients with painful knee osteoarthritis. Pain 2010; 149:573-581.

### Other references

6. Graven-Nielsen T, Wodehouse T, Langford RM, Arendt-Nielsen L, Kidd BL. Normalization of widespread hyperesthesia and facilitated spatial summation of deep-tissue pain in knee osteoarthritis patients after knee replacement. Arthritis Rheum. 2012 Sep;64(9):2907-16.
7. Aranda-Villalobos P, Fernández-de-Las-Peñas C, Navarro-Espigares JL, Hernández-Torres E, Villalobos M, Arendt-Nielsen L, Arroyo-Morales M. Normalization of widespread pressure pain hypersensitivity after total hip replacement in patients with hip osteoarthritis is associated with clinical and functional improvements. Arthritis Rheum. 2013 May;65(5):1262-70.
8. Suokas AK, Walsh DA, McWilliams DF, Condon L, Moreton B, Wylde V, Arendt-Nielsen L, Zhang W. Quantitative sensory testing in painful osteoarthritis: a systematic review and meta-analysis. Osteoarthritis Cartilage. 2012 Oct;20(10):1075-85.
9. Skou ST, Roos EM, Laursen MB, Rathleff MS, Arendt-Nielsen L, Simonsen OH, Rasmussen S. Total knee replacement plus physical and medical therapy or treatment with physical and medical therapy alone: a randomised controlled trial in patients with knee osteoarthritis (the MEDIC-study). BMC Musculoskelet Disord. 2012 May 9;13:67

## Claims

1. System for inducing at least compressional or shear force on a subject and evaluating pain modulation, spatial and/or temporal summation of pain, or descending inhibition of pain in the subject, said system comprising:
- a cuff unit comprising an inflatable cuff having at least two chambers or comprising at least two inflatable cuffs having one or more chambers each, each chamber having a coupling for connection to a compressor or other activator device;
- a compressor or other activator device arranged to provide inflation; and
- a computer unit with a control function, said control function directed to control of inflation rates and independent control of each chamber.

2. System according to claim 1, wherein at least one inflatable cuff of the system has more than two individual chambers.

3. System according to claim 1 or 2, wherein independent control of each chamber is independent inflation control and control of each chamber based on pressure, volume, tissue stress and/or tissue strain in a single inflation manner, in a repeated inflation manner and/or in an individual cuff manner.

4. System according to any of claims 1-3, wherein the inflatable cuff has several chambers of which one most distal chamber and one most proximal chamber are mounting chambers and one or more inner chamber(s) are stimulation chamber(s) or other activators integrated in the cuff.

5. Inflatable cuff for inducing at least compressional or shear force on a subject and evaluating pain modulation, spatial and/or temporal summation of pain, or descending inhibition of pain in the subject, said cuff comprising at least two separate chambers, wherein each chamber has a coupling for connection to a compressor or other activator device, and wherein each chamber is arranged for individual inflation and control thereof.

6. Inflatable cuff according to claim 5, wherein the cuff has at least a portion being made of a material which ensures compliance to adapt to anatomical structures.

7. Method for inducing at least compressional or shear force on a subject and evaluating pain modulation, spatial and/or temporal summation of pain, or descending inhibition of pain in the subject, said method comprising applying compressional or shear force pulses to the subject with a force pattern being uniform for pulses in the system according to any of claims 1-4.

8. Method for inducing at least compressional or shear force on a subject and evaluating pain modulation, spatial and/or temporal summation of pain, or descending inhibition of pain in the subject, said method comprising applying compressional or shear force pulses to the subject with an increasing force pattern over time via the inflatable cuff(s) in the system according to any of claims 1-4.

9. Method for inducing at least compressional or shear force on a subject and evaluating pain modulation, spatial and/or temporal summation of pain, or descending inhibition of pain in the subject, said method directed to assessing pain control in the subject, and wherein two separate cuffs in a system according to any of claims 1-4 are used, one or more cuff(s) on one site of a patient and the other one or more cuff(s) on another site of the patient, and wherein one or more of the cuffs are used to induce at least compressional or shear force on the subject and wherein the other one or more cuffs is used to measure the pain sensitivity and/or spatial/temporal summation of pain or descending inhibition of pain in the subject.

10. Method according to any of claims 7-9, wherein two or more separate cuffs are used and are inflated simultaneous or in predefined sequences.

11. Method according to any of claims 7-10, wherein two or more separate cuffs are used on different regions of the patient and wherein the two or more separate cuffs are controlled individually and in defined inflating sequences.

12. Use of a system according to any of claims 1-4, as a tool for diagnosis, for patient categorizing, for patient selecting, for treatment planning and/or for drug screening profiling.

13. Use of a system according to any of claims 1-4, to evaluate the pharmacological pain treatment of a patient.

14. Use of a system according to any of claims 1-4, for assessing tissue compliance.

15. Use of a system according to any of claims 1-4, for assessing descending pain control and assessing temporal/spatial summation in the same system.
